# EUROPEAN PATENT SPECIFICATION

(11) **EP 0 342 649 B1**
(45) Date of publication and mention of the grant of the patent: **30.11.1994**
(21) Application number: 89108874.2
(22) Date of filing: 17.05.1989
(51) Int. Cl.: C07D 401/04

(54) **Improved process for the preparation of 5-amino-7-(substituted amino)-quinoline-3-carboxylic acids**
Verfahren zur Herstellung von 5-Amino-7(substituierte amino)-chinolin-3-carbonsäure
Procédé de préparation des acides 5-amino-7-(amino substitué)-quinoléine-3-carboxyliques

(30) Priority: 18.05.1988 US 195580; 04.04.1989 US 331845
(43) Date of publication of application: 23.11.1989
(73) Proprietor: WARNER-LAMBERT COMPANY, Morris Plains New Jersey 07950 (US)
(72) Inventor: Wemple, James Norton, Holland Michigan 49424 (US)
(74) Representative: Mansmann, Ivo

(56) References cited:
- EP-A- 0 221 463
- EP-A- 0 242 789
- CHEMISCHE BERICHTE, vol. 101, 1968, pages 3623-3641; F. WEYGAND et al.:"Vergleichende Untersuchungen zur Abspaltung substit. Benzylreste vom Amidstickstoff und deren Kombinationsmöglichkeiten mit Urethanschutzgruppen"

## Description

### BACKGROUND OF THE INVENTION

The present invention is an improved process for the preparation of certain antibacterial agents described in European Patent Publications 172651 and 221463. The 5-amino-quinolones described in these publications have been shown to have excellent antibacterial activity against both gram-positive and gram-negative bacteria.

In European Publication 172651, the 5-amino-quinolones are prepared by reacting 5-amino-1-cyclopropyl-6,7,8-trifluoro-1,4-dihydro-4-oxo-quinoline-3-carboxylic acids with corresponding substituted amine derivatives to make 7-aminosubstituted-quinolones. This approach involves the complicated preparation of the above-mentioned 5-amino intermediate compound.

In U.S. Patent 4,795,751 a different approach to the synthesis of 5-amino-quinolones is described which involves treating a 7-substituted-amino-1-cyclopropyl-5,6,8-trifluoro-1,4-dihydro-4-oxoquinoline-3-carboxylic acid with ammonia or with benzylamine followed by removal of the benzyl group. Overall yields reported in this European publication vary from 50-60%.

The process described herein is a practical, cost-effective route to 5-amino-7-substituted-amino-quinolones in higher overall yields, e.g., 80-85%. The mild conditions used in each step permit formation of a higher purity product and only one step is required to remove both blocking groups on two different amino functional groups.

### SUMMARY OF THE INVENTION

Accordingly, the present invention is a process for the preparation of a hydrochloride salt of a compound of Formula I 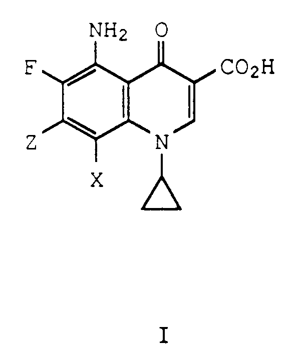
wherein Z is a group of the formula 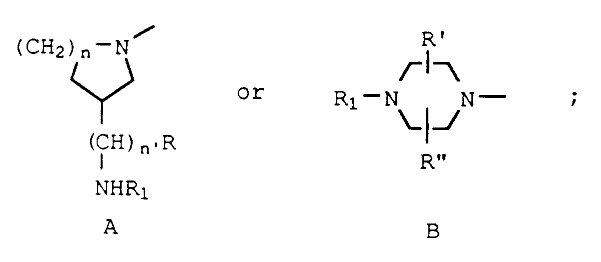
X is H, F or Cl; n is 1, 2, or 3; n′ is 0 or 1, and R, R₁, R′, and R˝ are each independently hydrogen or alkyl of one to three carbon atoms,
which comprises reacting a compound of Formula II 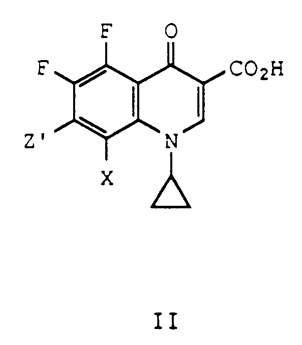
wherein Z' is a group of the formula 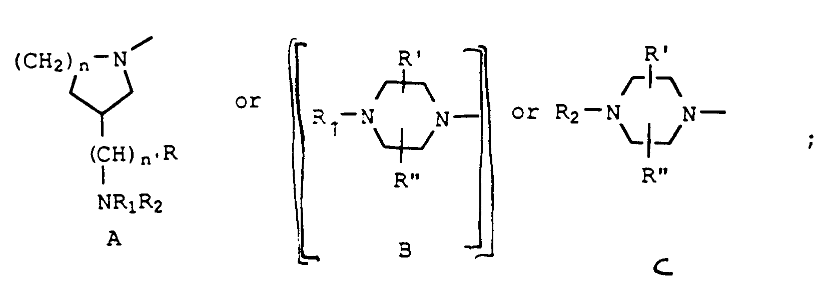
X, n, n', R, R₁, R', and R'' are as defined above, and R₂ is a protecting group capable of being removed under acid conditions, with a mono-, di-, or trialkoxybenzylamine in the presence of a base and in a polar, aprotic solvent at about 60 to about 150°C for about 2 to about 14 hours; and reacting the resulting compound of Formula III 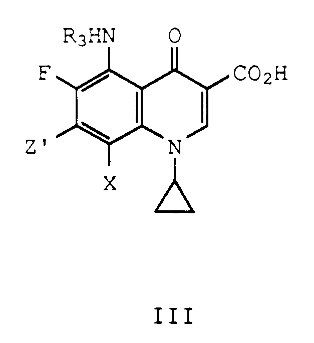
wherein Z', X, n, n', R, R₁, R', R'', and R₂ are as defined above and R₃ is a mono-, di-, or trialkoxybenzyl group, in which alk is one to three carbon atoms, with aqueous hydrochloric acid in the presence of an inert, water immiscible solvent at about 25 to about 100°C for about 3 to about 24 hours.

The product is then isolated by precipitating the hydrochloride salt of the compound of Formula I by treating the aqueous hydrochloric acid layer with a water miscible solvent.

### DESCRIPTION OF THE PREFERRED EMBODIMENTS

Compounds of Formula II are either known or, if new, may be prepared by known methods as described, for example, in U.S. Patent Nos.4,657,913 and 4,795,751 which are incorporated herein by reference.

The protecting group "R₂" in Formula II is one capable of being removed under acid conditions such as, for example, tert-butoxycarbonyl, benzyloxycarbonyl, p-methoxybenzyloxycarbonyl, p-methylbenzyloxycarbonyl, and the like. The preferred protecting group is tert-butoxycarbonyl.

Treatment of a compound of Formula II in the first step of the synthesis with a mono-, di-, or trialkoxybenzylamine, in which alk is one to three carbon atoms, includes particularly and preferably p-methoxybenzylamine, 3,4-dimethoxybenzylamine, 2,4-dimethoxybenzylamine or 2,4,6-trimethoxybenzylamine. Most preferred is p-methoxybenzylamine.

The reaction is carried out in the presence of a base such as, for example, triethylamine, N-ethylmorpholine, ethyldiisopropylamine, pyridine, or a related tertiary amine. Anhydrous potassium carbonate, sodium carbonate, sodium bicarbonate, or a related carbonate may also be used. The preferred base is triethylamine.

The polar, aprotic solvent may be dimethylsulfoxide, acetonitrile, sulfolane, N,N-dimethylacetamide, N-methylpyrrolidine, N,N-dimethylformamide, pyridine, or a relative thereof. The preferred solvent is dimethylsulfoxide.

The second step involves removal of both blocking groups to afford the desired antibacterial in the form of its hydrochloride salt. The reaction is carried out in hydrochloric acid in the presence of an inert, water immiscible solvent such as, for example, toluene, heptane, n-butyl ether, chlorobenzene, methylene chloride or a relative thereof. The preferred solvent is toluene.

The isolation of the final produced as a hydrochloride salt is carried out by precipitating the product out of the aqueous hydrochloric acid layer by adding thereto a water miscible solvent such as, for example, isopropyl alcohol, n-propyl alcohol, ethyl alcohol, or other alcoholic solvents. The preferred solvent is isopropyl alcohol.

Preferred compounds of Formula I prepared by the process of the present invention and isolated as their respective hydrochloride salts are those wherein Z is as defined above; X is H, F or Cl; n is 1 or 2; n′ is 0 or 1; R is hydrogen or methyl; R₁ is hydrogen, methyl or ethyl, and R′ and R˝ are each independently hydrogen or methyl.

More preferred are the compounds of Formula I, wherein Z is as defined above; X is F; n is 1 or 2; n′ is 0 or 1; R is hydrogen; R₁ is hydrogen, methyl or ethyl, and R′ and R˝ are each hydrogen or methyl.

Particularly valuable are:
5-amino-7-(3-amino-1-pyrrolidinyl)-8-chloro-1-cyclopropyl-6-fluoro-1,4-dihydro-4-oxoquinoline-3-carboxylic acid;
5-amino-8-chloro-1-cyclopropyl-6-fluoro-1,4-dihydro-7-[3-[(methylamino)methyl]-1-pyrrolidinyl]-4-oxoquinoline-3-carboxylic acid;
5-amino-7-(3-amino-1-pyrrolidinyl)-1-cyclopropyl-6,8-difluoro-1,4-dihydro-4-oxoquinoline-3-carboxylic acid;
5-amino-1-cyclopropyl-6,8-difluoro-7-[3-(ethylamino)methyl-1-pyrrolidinyl)]-1,4-dihydro-4-oxoquinoline-3-carboxylic acid;
5-amino-1-cyclopropyl-6,8-difluoro-7-[3-(aminomethyl)-1-pyrrolidinyl]-1,4-dihydro-4-oxoquinoline-3-carboxylic acid, and
5-amino-1-cyclopropyl-6,8-difluoro-1,4-dihydro-7-(cis-3,5-dimethylpiperazin-1-yl)-4-oxoquinoline-3-carboxylic acid.

The compounds of the invention can exist in unsolvated as well as solvated forms, including hydrated forms. In general, the solvated forms, including hydrated forms and the like are equivalent to the unsolvated forms for purposes of the invention.

Certain compounds of the invention may exist in optically active forms. The pure D isomer, pure L isomer as well as mixtures thereof; including the racemic mixtures, are contemplated by the invention. Additional asymmetric carbon atoms may be present in a substituent such as an alkyl group. All such isomers as well as mixtures thereof are intended to be included in the invention.

The following examples illustrate the inventors' preferred methods for preparing the compounds of the invention.

### PREPARATION OF STARTING MATERIAL

### EXAMPLE A

### 7-[3-(tert-Butoxycarbonylamino)pyrrolidin-1-yl]-1-cyclopropyl-5,6,8-trifluoro-1,4-dihydro-4-oxoquinoline-3-carboxylic acid.

1-Cyclopropyl-5,6,7,8-tetrafluoro-1,4-dihydro-4-oxoquinoline-3-carboxylic acid (30.1 g) and 3-(tert-butoxycarbonylamino)pyrrolidine (20.5 g) were treated with N,N-dimethylformamide (200 ml) and the mixture heated to 50-60°C. Triethylamine (20.5 g) was added dropwise over 15 minutes. The mixture was then stirred at 50-55°C for four hours. Acetonitrile (200 ml) was then added and the mixture heated to 75°C and then allowed to cool to 15°C and filtered. The solid product was washed with acetonitrile (2 x 100 ml) and then reslurried with 300 ml of acetonitrile (2 x 100 ml). The mixture was filtered and washed with more acetonitrile (2 x 100 ml). The product was vacuum dried to give the titled compound (41.5 g; 89%); mp 244-246°C.

### EXAMPLE B

### 1-Cyclopropyl-5,6,8-trifluoro-1,4-dihydro-7-(cis-3,5-dimethylpiperazin-1-yl)-4-oxoquinoline-3-carboxylic acid.

1-Cyclopropyl-5,6,7,8-trifluoro-1,4-dihydro-4-oxoquinoline-3-carboxylic acid (14.5 g) and cis-2,6-dimethylpiperazine (5.6 g) were charged to a flask. N,N-Dimethylformamide (100 ml) was then added at room temperature followed by triethylamine (4.8 g). The resulting mixture was then allowed to stir at room temperature for 84 hours. The solid product was collected by filtration and washed with acetonitrile (2 x 25 ml) and finally vacuum dried to give the title compound (17.75 g, 93%, HPLC: 95.5%). This material was used directly in the next reaction step, see Example 5. A small portion was recrystallized from acetonitrile; mp 243-255°C (dec; HPLC: 96.9%).

| Anal. for C₁₉H₂₀F₃N₃O₃ - 1/8 H₂O: | | | |
|---|---|---|---|
| Calc.: | C, 57.39; | H, 5.13; | N, 10.57. |
| Found: | C, 57.17; | H, 5.19; | N, 10.66. |

### EXAMPLE 1

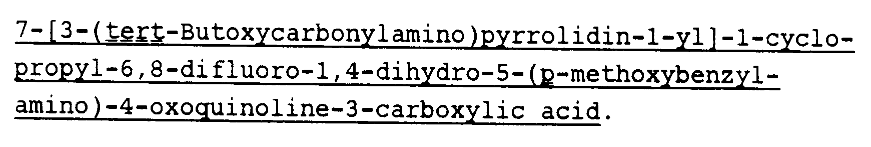

7-[3-(tert-Butoxycarbonylamino)pyrrolidin-1-yl]-1-cyclopropyl-5,6,8-trifluoro-1,4-dihydro-4-oxoquinoline-3-carboxylic acid (190 g) was added to dimethylsulfoxide (475 ml). p-Methoxybenzylamine (105.5 g) was added to the mixture which was then heated to 75-80°C. Triethylamine (257.5 ml) was then added slowly. The mixture was heated with stirring at reflux (90-100°C) for 4.5 hours and then diluted with acetonitrile (1.05 L). The mixture was cooled with stirring to 15°C where it was maintained for one hour. The crystals were collected and washed with acetonitrile (2 x 650 ml) followed by water (2 L). The solid product was then treated with 0.5% HCl (1800 ml) and the mixture stirred for 30 minutes at room temperature. It was filtered and the solid washed again with water (2 L) and vacuum dried to give the title compound (211 g, 89%); mp 207-209°C (dec); HPLC: 99.6%.

### EXAMPLE 2

### 5-Amino-7-(3-aminopyrrolidin-1-yl)-1-cyclopropyl-6,8-difluoro-1,4-dihydro-4-oxoquinoline-3-carboxylic acid hydrochloride monohydrate.

7-[3-(tert-Butoxycarbonylamino)pyrrolidin-1-yl]-1-cyclopropyl-6,8-difluoro-1,4-dihydro-5-(p-methoxybenzylamino)-4-oxoquinoline-3-carboxylic acid (8.6 g) was treated with toluene (30 ml) and the mixture stirred at room temperature. 36% hydrochloric acid (55 ml) was then added over 15 minutes with gas evolution. The solution was warmed to 30-40°C where it was maintained for seven hours. The aqueous layer was separated from the toluene layer. The aqueous layer was then treated with isopropyl alcohol (200 ml) and the resulting mixture cooled to 10°C. The crystals were collected, washed with isopropyl alcohol (2 x 40 ml) and vacuum dried to give the title compound (5.5 g; 90%): mp >300°C (dec); HPLC: 99.6%.

### EXAMPLE 3

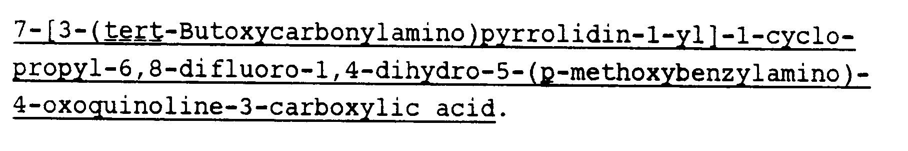

7-[3-(tert-Butoxycarbonylamino)pyrrolidin-1-yl]-1-cyclopropyl-5,6,8-trifluoro-1,4-dihydro-4-oxoquinoline-3-carboxylic acid (38.0 kg) and dimethylsulfoxide (95 L) were charged to a 100-gallon stainless steel still. p-Methoxybenzylamine (21.1 kg) was charged to this mixture at room temperature. The batch was heated to 75°C under a nitrogen atmosphere with stirring. Triethylamine (37.4 kg) was then added at a rate of 0.8 to 1.2 L/min while maintaining the batch temperature below 75°C. The mixture was then heated to reflux where it was maintained for 4.5 hours. The mixture was cooled to 80°C and acetonitrile (210 L) added. The batch was stirred and cooled over 2.5 hours to 10°C. The product was collected on a centrifuge and the yellow crystals washed on the centrifuge with acetonitrile (2 x 130 L) followed by water (400 L). The product was removed from the centrifuge and reslurried in the 100-gallon still with 355 L demineralized water and 6 L 36% hydrochloric acid. This mixture was stirred for one hour at 26°C. The product was collected on the centrifuge, washed with demineralized water (200 L) and then vacuum dried at 50-55°C to give the above titled compound (42.7 kg, 90%): mp 207-209°C (dec.); HPLC: 100%.

### EXAMPLE 4

### 5-Amino-7-(3-aminopyrrolidin-yl)-1-cyclopropyl-6,8-difluoro-1,4-dihydro-4-oxoquinoline-3-carboxylic acid hydrochloride monohydrate.

7-[3-(tert-Butoxycarbonylamino)pyrrolidin-1-yl]-1-cyclopropyl-6,8-difluoro-1,4-dihydro-5-(p-methoxybenzylamino)-4-oxoquinoline-3-carboxylic acid (42.65 kg) and toluene (150 L) were charged to a 200-gallon glass-lined still. The mixture was stirred at 25°C under a nitrogen atmosphere and 300 L of 36% HCl was added over a 105-minute period. The resulting mixture was then heated with stirring at 33-42°C for 6.5 hours. The lower aqueous layer was filtered through a 0.45-micron Pall filter and the filtrate transferred into a 400-gallon glass-lined reactor. Isopropyl alcohol was charged to the reactor while maintaining the temperature below 36°C. The resulting mixture was stirred for 110 minutes while cooling to 13°C. The product was collected on the centrifuge and the yellow crystals washed with isopropyl alcohol (286 L). The solid was removed from the centrifuge and reslurried with 400 L isopropyl alcohol at 15-30°C for 40 minutes in the 400-gallon still. The yellow crystals were collected again on the centrifuge and washed with isopropyl alcohol (159 L). The product was vacuum dried at 35-50°C for three days to give the above titled compound (25.15 kg, 82%): mp >300°C (dec); HPLC: 99.4%. A second crop was isolated from the mother liquors using a Niagara filter: 3.0 kg (10%); HPLC: 99.4%.

### EXAMPLE 5

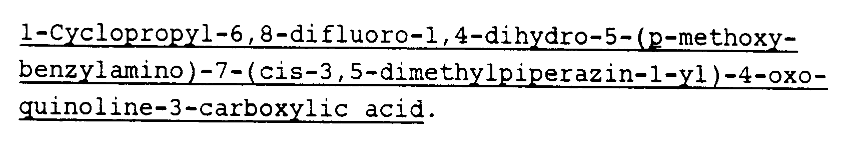

1-Cyclopropyl-5,6,8-trifluoro-1,4-dihydro-7-(cis-3,5-dimethylpiperazin-1-yl)-4-oxoquinoline-3-carboxylic acid (8.25 g) and p-methoxybenzylamine (5.25 g) were charged to a flask followed by DMSO (25 ml). This mixture was heated with stirring to 35°C, at which point triethylamine (9.5 g) was added. The mixture was then warmed with continued stirring to 90°C-95°C where it was maintained for 4.5 hours. Acetonitrile (55 ml) was then added and the mixture allowed to cool overnight to room temperature. The crystals were collected and washed with acetonitrile (50 ml) and vacuum dried to give the title compound (9.0 g, 84%, HPLC: 99.78%). This material contained some residual p-methoxybenzylamine according to NMR analysis, however, it was used without further purification in the next step, Example 6. A small portion (0.5 g) was recrystallized from a mixture of acetonitrile (35 ml) and acetic acid (0.5 g) to remove the p-methoxbenzylamine. This gave the mono acetic acid salt of the title compound, mp 150.5-177°C; HPLC: 97.42%.

| Anal. for C₂₇H₃₀F₂N₄O₄-CH₃CO₂H-1/2 H₂O: | | | |
|---|---|---|---|
| Calc.: | C, 59.88; | H, 6.06; | N, 9.64. |
| Found: | C, 59.79; | H, 5.94; | N, 9.61. |

### EXAMPLE 6

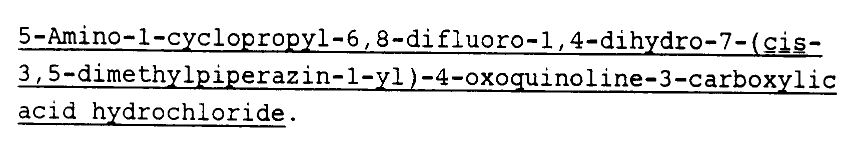

1-Cyclopropyl-6,8-difluoro-1,4-dihydro-5-(p-methoxybenzylamino)-7-(cis-3,5-dimethylpiperazin-1-yl)-4-oxoquinoline-3-carboxylic acid (4.2 g) was treated with toluene (15 ml) and the mixture stirred at room temperature. Hydrochloric acid (36%, 30 ml) was added and the mixture stirred and heated to 45-50°C where it was maintained for six hours. The organic phase was separated and the aqueous layer treated with isopropyl alcohol (90 ml). A yellow solid began to form. The mixture was stirred and cooled to 25°C over 30 minutes and the solid collected. The mixture was filtered and the solid washed with isopropyl alcohol (2 x 15 ml) and vacuum dried to give 5-amino-1-cyclopropyl-6,8-difluoro-1,4-dihydro-7-(cis-3,5-dimethylpiperazin-1-yl)-4-oxoquinoline-3-carboxylic acid hydrochloride (2.95 g, 84%), mp >320°C; HPLC: 99.69%.

### COMPARATIVE EXAMPLE

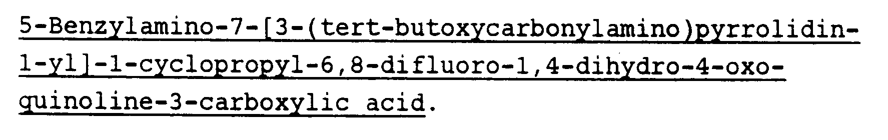

7-[3-(tert-Butoxycarbonylamino)pyrrolidin-1-yl]-1-cyclopropyl-5,6,8-trifluoro-1,4-dihydro-4-oxoquinoline-3-carboxylic acid (10.0 g) and benzylamine (5.5 g) were stirred with dimethylsulfoxide (25 g). Triethylamine (7.0 g) was added and the mixture heated to reflux where it was maintained for seven hours. The mixture was then concentrated under reduced pressure to remove the dimethylsulfoxide solvent. The residue was treated with acetonitrile with stirring at 40-50°C. The resulting mixture was stirred and cooled to 10°C, filtered, and the crystals washed with acetonitrile (2 x 50 ml). After drying, the solid was reslurried with 0.5 N HCl (200 ml) and the mixture stirred 30 minutes at room temperature. The solid was collected, washed with water (2 x 75 ml) and vacuum dried to give the above titled compound (10.7 g; 89%): mp 174-176°C; HPLC: 99.7%.

### 5-Amino-7-(3-aminopyrrolidin-1-yl)-1-cyclopropyl-6,8-difluoro-1,4-dihydro-4-oxoquinoline-3-carboxylic acid hydrochloride monohydrate.

5-Benzylamino-7-[3-(tert-butoxycarbonylamino)pyrrolidin-1-yl]-1-cyclopropyl-6,8-difluoro-1,4-dihydro-4-oxoquinoline-3-carboxylic acid (4.1 g) was treated with toluene (30 ml) and the mixture stirred at room temperature. 36% Hydrochloric acid (27 ml) was added over 15 minutes with gas evolution. The solution was then heated with stirring to 68-72°C where it was maintained for six hours. The layers were separated and the warm aqueous layer treated with isopropyl alcohol (125 ml). The resulting mixture was cooled with stirring to 10°C and filtered. The product was washed with isopropyl alcohol (2 x 35 ml) and vacuum dried to give the above titled compound (2.7 g; 87%): mp >300°C (dec); HPLC: 79%. The same reaction was carried out at 63-67°C for four hours. This resulted in a higher purity product (HPLC: 94%) but a lower yield (71%).

## Claims

1. A process for the preparation of a hydrochloride salt of a compound of the formula 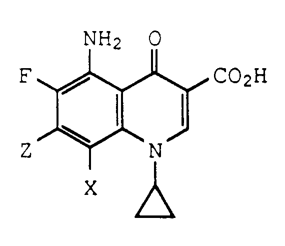 wherein Z is a group of the formula 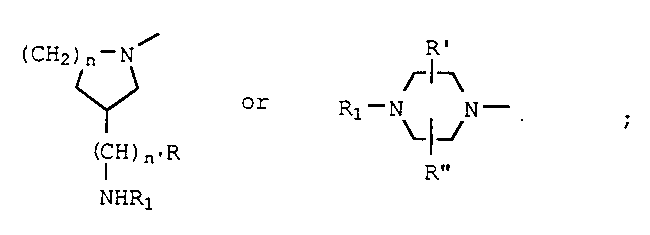 X is H, F or Cl; n is 1, 2, or 3; n′ is 0 or 1, and R, R₁, R′, and R˝ are each independently hydrogen or alkyl of one to three carbon atoms, which comprises reacting a compound of the formula 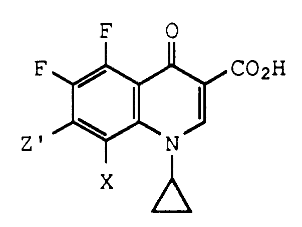 wherein Z′ is a group of the formula 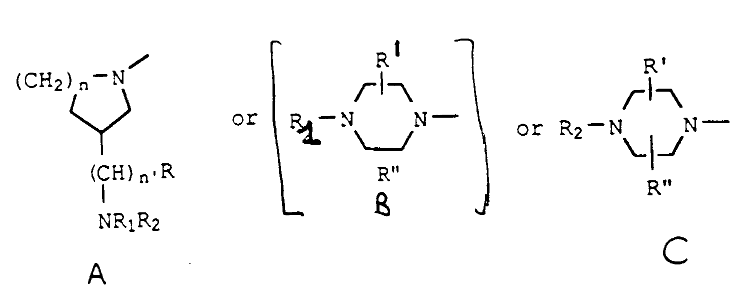 R₂ is a protecting group capable of being removed under acid conditions, with a mono-, di- or trialkoxybenzylamine, in which alk is one to three carbon atoms, in the presence of a base and in a polar, aprotic solvent at about 60 to about 150°C for about 2 to about 14 hours; and reacting the resulting compound of the formula 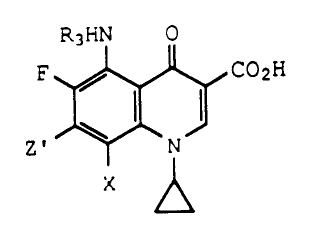 wherein R₃ is a mono-, di- or trialkoxybenzyl group, in which alk is one to three carbon atoms, with aqueous hydrochloric acid in the presence of an inert, water immiscible solvent at about 25 to about 100°C for about 3 to about 24 hours.

2. The process of Claim 1, wherein R₂ is selected from the group consisting of tert-butoxycarbonyl, benzyloxycarbonyl, p-methoxybenzyloxycarbonyl and p-methylbenzyloxycarbonyl.

3. The process of Claim 2, wherein R₂ is tert-butoxycarbonyl.

4. The process of Claim 1, wherein R₃ is selected form the group consisting of p-methoxybenzyl, 3,4-dimethoxybenzyl, 2,4-dimethoxyphenyl, and 2,4,6-trimethoxybenzyl.

5. The process of Claim 4, wherein R₃ is p-methoxybenzyl.

6. The process of Claim 1, wherein the base is triethylamine, N-ethylmorpholine, ethyldiisopropylamine, pyridine, anhydrous potassium carbonate or sodium carbonate.

7. The process of Claim 6, wherein the base is triethylamine.

8. The process of Claim 1, wherein the polar, aprotic solvent is dimethylsulfoxide, acetonitrile, sulfolane, N,N-dimethylacetamide, N-methylpyrrolidine, N,N-dimethylformamide or pyridine.

9. The process of Claim 8, wherein the polar, aprotic solvent is dimethylsulfoxide.

10. The process of Claim 1, wherein the inert water immiscible solvent is toluene, heptane, n-butyl ether, chlorobenzene, or methylene chloride.

11. The process of Claim 10, wherein the inert water immiscible solvent is toluene.

12. The process of Claim 1, wherein X is H, F or Cl; n is 1 or 2; n′ is 0 of 1; R is hydrogen or methyl; R₁ is hydrogen, methyl or ethyl, and R′ and R˝ are each independently hydrogen or methyl.

13. The process of Claim 12, wherein R₂ is tert-butoxycarbonyl and R₃ is p-methoxybenzyl.

14. The process of Claim 12, wherein X is F; n is 1 or 2; n′ is 0 or 1; R is hydrogen; R₁ is hydrogen, methyl or ethyl, and R′ and R˝ are each independently hydrogen or methyl.

15. The process of Claim 14, wherein R₂ is tert-butoxycarbonyl and R₃ is p-methoxybenzyl.

16. The process of Claim 1 and for the preparation of the hydrochloride salt of 5-amino-7-(3-amino-1-pyrrolidinyl)-1-cyclopropyl-6,8-difluoro-1,4-dihydro-4-oxo-3-quinolinecarboxylic acid.

17. The process of Claim 1 and for the preparation of the hydrochloride salt of 5-amino-1-cyclopropyl-6,8-difluoro-1,4-dihydro-7-(cis-3,5-dimethylpiperazin-1-yl)-4-oxoquinoline-3-carboxylic acid.

18. The process of Claim 1, wherein the hydrochloride salt is isolated by removing the aqueous hydrochloric acid layer and precipitating the salt therefrom by adding a water miscible solvent.

19. The process of Claim 18, wherein the water miscible solvent is isopropyl alcohol.

## Patentansprüche

1. Verfahren zur Herstellung eines Hydrochlorid-Salzes einer Verbindung der Formel 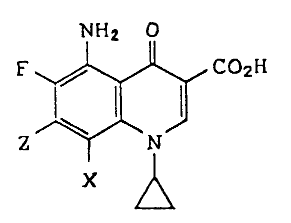 worin Z eine Gruppierung der Formel 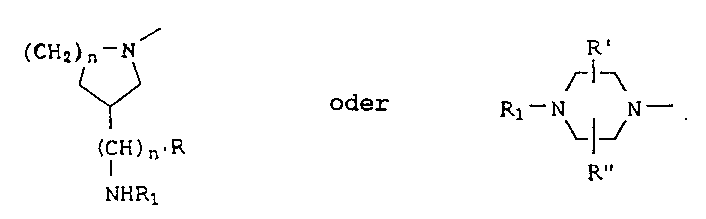 darstellt, worin
X H, F oder Cl;
n 1, 2 oder 3;
n' Null oder 1; und
R, R₁, R' und R'' jeweils unabhängig voneinander Wasserstoff oder Alkyl mit ein bis drei Kohlenstoffatomen
bedeuten, wobei das Verfahren die folgenden Verfahrensstufen umfasst:
Umsetzung einer Verbindung der Formel 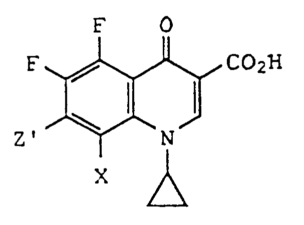 worin
Z' für eine Gruppe der Formel A, B oder C 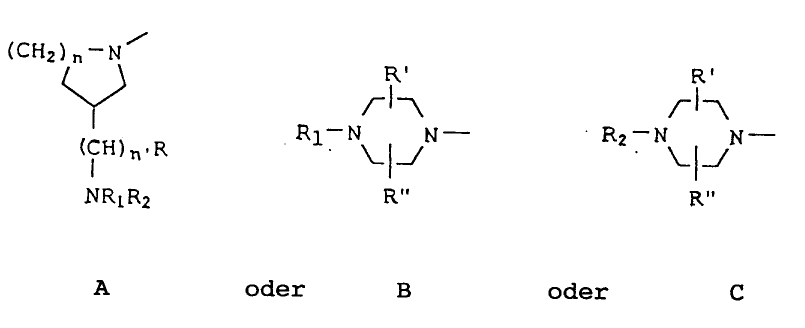
steht; und
R₂ eine unter sauren Bedingungen entfernbare Schutzgruppe
bedeutet, mit einem Mono-, Di- oder Trialkoxybenzylamin, wobei alk ein bis drei Kohlenstoffatome aufweist, in Gegenwart einer Base und in einem polaren, aprotischen Lösungsmittel bei etwa 60 bis etwa 150°C während etwa 2 bis etwa 14 Stunden; und
Umsetzung der entstandenen Verbindung der Formel 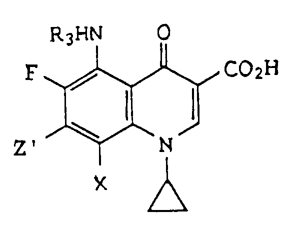 worin R₃ eine Mono-, Di- oder Trialkoxybenzylgruppe, wobei alk ein bis drei Kohlenstoffatome enthält, bedeutet, mit wässriger Chlorwasserstoffsäure in Gegenwart eines inerten mit Wasser nichtmischbaren Lösungsmittels bei etwa 25 bis etwa 100°C im Laufe von etwa 3 bis etwa 24 Stunden.

2. Verfahren nach Anspruch 1, worin R₂ aus der aus tert-Butoxycarbonyl, Benzyloxycarbonyl, p-Methoxybenzyloxycarbonyl und p-Methylbenzyloxycarbonyl bestehenden Gruppe ausgewählt ist.

3. Verfahren nach Anspruch 2, worin R₂ für tert-Butoxycarbonyl steht.

4. Verfahren nach Anspruch 1, worin R₃ aus der aus p-Methoxybenzyl, 3,4-Dimethoxybenzyl, 2,4-Dimethoxyphenyl und 2,4,6-Trimethoxybenzyl bestehenden Gruppe ausgewählt ist.

5. Verfahren nach Anspruch 4, worin R₃ für p-Methoxybenzyl steht.

6. Verfahren nach Anspruch 1, bei dem als Base Triethylamin, N-Ethylmorpholin, Ethyldiisopropylamin, Pyridin, wasserfreies Kaliumcarbonat oder Natriumcarbonat verwendet wird.

7. Verfahren nach Anspruch 6, bei dem als Base Triethylamin verwendet wird.

8. Verfahren nach Anspruch 1, bei dem als polares, aprotisches Lösungsmittel Dimethylsulfoxid, Acetonitril. Sulfolan, N,N-Dimethylacetamid, N-Methylpyrrolidin, N,N-Dimethylformamid oder Pyridin verwendet wird.

9. Verfahren nach Anspruch 8, bei dem als polares, aprotisches Lösungsmittel Dimethylsulfoxid verwendet wird.

10. Verfahren nach Anspruch 1, bei dem als inertes, mit Wasser nicht mischbares Lösungsmittel Toluol, Heptan, n-Butyläther, Chlorbenzol oder Methylenchlorid verwendet wird.

11. Verfahren nach Anspruch 10, bei dem als inertes mit Wasser nicht mischbares Lösungsmittel Toluol verwendet wird.

12. Verfahren nach Anspruch 1 zur Herstellung einer Verbindundung, worin X für H, F oder Cl steht; n 1 oder 2 bedeutet; n' Null oder 1 ist; R für Wasserstoff oder Methyl steht; R₁ Wasserstoff, Methyl oder Ethyl darstellt und R' und R'' jeweils unabhängig voneinander Wasserstoff oder Methyl bedeuten.

13. Verfahren nach Anspruch 12, worin R₂ tert-Butoxycarbonyl und R₃ p-Methoxybenzyl bedeuten.

14. Verfahren nach Anspruch 12 zur Herstellung einer Verbindung, worin X für F steht; n 1 oder 2 bedeutet; n' Null oder 1 darstellt; R Wasserstoff ist; R₁ Wasserstoff, Methyl oder Ethyl ist und R' und R'' jeweils unabhängig voneinander Wasserstoff oder Methyl bedeuten.

15. Verfahren nach Anspruch 14, worin R₂ tert-Butoxycarbonyl und R₃ p-Methoxybenzyl bedeuten.

16. Verfahren nach Anspruch 1 und zur Herstellung des Hydrochlorid-Salzes von 5-Amino-7-(3-amino-1-pyrrolidinyl)-1-cyclopropyl-6,8-difluor-1,4-dihydro-4-oxo-3-chinolincarbonsäure.

17. Verfahren nach Anspruch 1 und zur Herstellung des Hydrochlorid-Salzes von 5-Amino-1-cyclopropyl-6,8-difluor-1,4-dihydro-7-(*cis*-3,5-dimethylpiperazin-1-yl)-4-oxochinolin-3-carbonsäure.

18. Verfahren nach Anspruch 1, bei dem das Hydrochlorid-Salz durch Entfernen der wässerigen chlorwasserstoffsauren Schicht und Ausfällen des Salzes aus derselben durch Zusatz eines mit Wasser mischbaren Lösusngsmittels isoliert wird.

19. Verfahren nach Anspruch 18, bei dem das mit Wasser mischbare Lösungsmittel Isopropylalkohol ist.

## Revendications

1. Procédé de préparation d'un chlorhydrate de composé d'un composé de formule 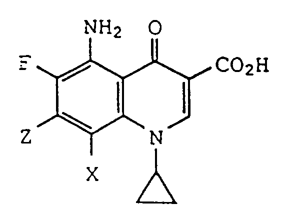 dans laquelle Z est un groupe de formule 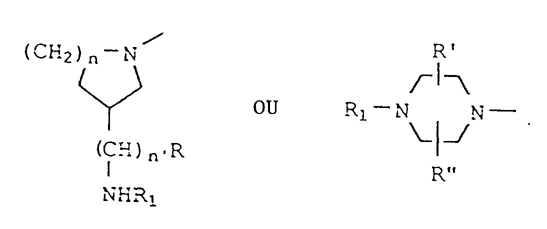 X est H, F ou Cl; n est 1, 2, ou 3; n' est 0 ou 1, et R, R₁, R', et R'' sont chacun indépendamment les uns des autres un atome d'hydrogène ou un groupe alkyle ayant 1 à 3 atomes de carbone, qui comprend la réaction d'un composé de formule 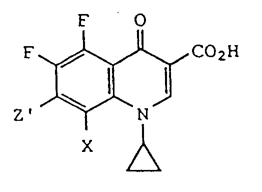 dans laquelle Z' est un groupe de formule 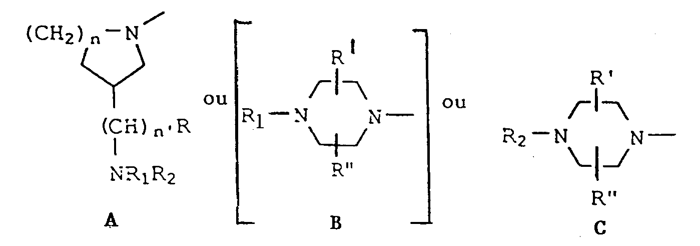 R₂ est un groupe protecteur susceptible d'être éliminé dans des conditions acides,
avec une mono-, di-, ou trialcoxybenzyl-amine dans laquelle la partie alkyle est un groupe ayant 1 à 3 atomes de carbone, en présence d'une base et dans un solvant polaire aprotique à une température d'environ 60 à environ 150°C pendant environ 2 à environ quatorze heures;
et la réaction du composé de formule 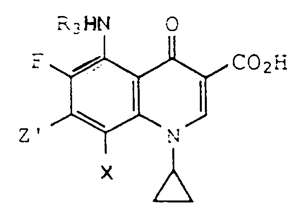 dans laquelle R₃ est un groupe mono-, di-, ou trialcoxybenzyle, dans lequel la partie alkyle est un groupe comprenant 1 à 3 atomes de carbone,
avec une solution acqueuse d'acide chlorhydrique en présence d'un solvant inerte immiscible à l'eau à une température d'environ 25 à 100°C pendant d'environ 3 à environ 24 heures.

2. Le procédé selon la revendication 1 dans lequel R₂ est choisi dans le groupe consistant en tert-butoxycarbonyle, benzyloxycarbonyle, p-méthoxybenzyloxycarbonyle et p-méthylbenzyloxycarbonyle.

3. Le procédé selon la revendication 2 dans lequel R₂ est un groupe tert-butoxycarbonyle.

4. Le procédé selon la revendication 1 dans lequel R₃ est choisi dans le groupe consistant en groupe p-methoxybenzyle, 3,4-diméthoxybenzyle, 2,4-diméthoxyphényel, et 2,4,6-trimethoxybenzyel.

5. Le procédé selon la revendication 4, dans lequel R₃ est un groupe p-méthoxybenzyle.

6. Le procédé selon la revendication 1, dans lequel la base est la triéthylamine, la N-éthylmorpholine, l'éthyldiisopropylamine, la pyridine, le carbonate de sodium ou le carbonate de potassium anhydre

7. Le procédé selon la revendication 6, dans lequel la base est la triéthylamine.

8. Le procédé selon la revendication 1, dans lequel le solvant polaire aprotique est le diméthylsulfoxyde, l'acétonitrile, le sulfolane, le N,N-diméthylacétamide, la N-méthylpyrrolidine, la N,N-dimethylformiamide ou la pyridine.

9. Le procédé selon la revendication 8, dans lequel le solvant polaire aprotique est le diméthylsulfoxyde.

10. Le procédé selon la revendication 1, dans lequel le solvant inerte immiscible à l'eau est le toluène, l'heptane, l'éther n-butylique, le chlorobenzène, ou le chlorure de méthylène.

11. Le procédé selon la revendication 10, dans lequel le solvant inerte immiscible à l'eau est le toluène.

12. Le procédé selon la revendication 1, dans lequel X est H, F ou Cl; n est 1 ou 2; n' est 0 ou 1; R est un atome d'hydrogène ou un groupe méthyle; R₁ est un atome d'hydrogène, un groupe méthyle ou éthyle et R' et R'' sont chacun indépendamment l'un de l'autre un atome d'hydrogène ou un groupe méthyle.

13. Le procédé selon la revendication 12, dans lequel R₂ est un groupe tert-butoxycarbonyle et R₃ est un groupe p-methoxybenzyle.

14. Le procédé selon la revendication 12, dans lequel X est F; n est 1 ou 2; n' est 0 ou 1; R est un atome d'hydrogène; R₁ est un atome d'hydrogène, un groupe méthyl ou éthyle, et R' et R'' sont chacun indépendamment l'un de l'autre un atome d'hydrogène ou un groupe méthyle.

15. Le procédé selon la revendication 14, dans lequel R₂ est un groupe tert-butoxycarbonyle et R₃ est un groupe p-méthoxybenzyle.

16. Le procédé selon la revendication 1 et pour la préparation du chlorhydrate de l'acide 5-amino-7-(3-amino-1-pyrrolidinyl)-1-cyclopropyl-6,8-difluoro-1,4-dihydro-4-oxo-3-quinoléine-3-carboxylique.

17. Le procédé selon la revendication 1 et pour la préparation du chlorhydrate de l'acide 5-amino-1-cyclopropyl-6,8-difluoro-1,4-dihydro-7-(cis-3,5-diméthylpipérazine-1-yl)-4-oxoquinoléine-3-carboxylique.

18. Le procédé selon la revendication 1, dans lequel le chlorhydrate est isolé par élimination de la couche d'acide chlorhydrique aqueuse et précipitation du sel à partir de celle-ci par addition d'un solvant miscible à l'eau.

19. Le procédé selon la revendication 18 lequel le solvant miscible à l'eau est l'alcool isopropylique.
